# EUROPEAN PATENT SPECIFICATION

(11) **EP 1 539 036 B1**
(45) Date of publication and mention of the grant of the patent: **03.11.2010**
(21) Application number: 03751968.3
(22) Date of filing: 03.09.2003
(51) Int. Cl.: A61F 2/92

(54) **STENT WITH MULTIPLE HELIX CONSTRUCTION**
STENT MIT SPIRALFÖRMIGER STRUKTUR
STENT AVEC STRUCTURE MULTI-HELICOIDALE

(30) Priority: 13.09.2002 US 242999
(43) Date of publication of application: 15.06.2005
(73) Proprietor: Gore Enterprise Holdings, Inc., Newark, DE 19714-9206 (US)
(72) Inventor: CULLY, Edward, H., Flagstaff, AZ 86004 (US); VONESH, Michael, J., Flagstaff, AZ 86004 (US)
(74) Representative: Shanks, Andrew
(86) International application number: PCT/US2003/027541
(87) International publication number: WO 2004/024033

(56) References cited:
- EP-A1- 0 806 190
- WO-A-00/42945
- US-A- 6 096 072
- US-A1- 2002 123 796

## Description

### BACKGROUND OF THE INVENTION

### 1. Field of the Invention

The present invention relates to medical devices and more particularly to medical devices that are designed to be inserted endoluminally in a body.

### 2. Description of Related Art

Recent developments in medicine have emphasized minimally invasive surgical procedures. It is common today for medical instruments to be remotely inserted into a patient's body through small, sometimes percutaneous, incisions and entire operations performed remotely using fluoroscopic, radiographic, ultrasonic, angioscopic, or other visualization techniques.

These remote techniques are regularly employed today in a variety of vascular procedures, including treatments for coronary artery disease or other vascular obstructions (e.g., balloon angioplasty and/or stenting), repair of aortic or other vascular aneurysms, creation of various vascular shunts, repair of heart defects, correction of other duct problems in the body, etc. Despite tremendous advancements in the area of minimally invasive interventions, additional improvements are believed possible, and are likely necessary to fully exploit the potential of this technology.

Specifically, it is common today for expandable stent devices to be placed in a vessel to help maintain flow through the vessel or to prevent fluid from filling an aneurysm or from leaking through a tear or other opening in the vessel wall. Stents for these procedures may be formed from a plastically deformable material that is enlarged in place within the vessel (such as through use of an inflatable balloon), or through an elastic or springy material that allows the stent to self-expand in place once a constraint mechanism is removed from a compacted stent. In either case, the stent may include a covering on one or both of its inner or outer surfaces to prevent fluid flow from passing through the interstices of the stent and/or prevent cell ingrowth through the stent structure.

A wide variety of stent designs have been proposed to provide various beneficial properties. Many stents are formed from wire material that is wound and sometimes welded or otherwise joined into desired patterns. Alternatively, stents can be formed from continuous sheets or tubes that are then cut and formed into the desired stent pattern. Typically, both of these manufacturing techniques yield stent designs that fall into a couple basic forms.

A first common design for stents is to have an essentially helical design whereby a single stent element can be defined as extending helically around a longitudinal axis from one end of the stent to the other. Usually the helical stent element includes an undulating (e.g., "zigzag") or other expandable pattern along its length. This design is particularly popular with wire-formed stents since it allows the stent to be formed from a single length of wire.

A second common design for stents is for the stent to comprise a series of discrete "ring" elements oriented essentially perpendicular to the longitudinal axis of the stent. The discrete ring elements are normally attached together by a series of one or more "connectors" or "bridges" extending between the rings. Again, the ring elements are usually formed with some form of undulating, diamond, serpentine, sinusoidal, or similar expandable pattern to allow compaction and/or expansion of the stent. By altering the shape and placement of the bridge elements it has been demonstrated that flexibility of the stent and its expansion properties can be tailored to address desired placement and operational specifications. Due to the complexity of many of the ring-and-bridge designs and the desire to avoid onerous forming and welding procedures, this design is most commonly employed with stents formed from a continuous tube or sheet of material that is cut into the desired pattern. A variation of this second type of stent is the so-called "closed-cell" design, typified by the J & J/Cordis Crown Stent and Medinol NIR stent.

While many of the existing stent designs function quite well for their intended purposes, it is believed that further improvements are possible. For example, with both of the above described common forms of stent designs it is often difficult to control the degree of shortening of the stent between its small delivery diameter and its enlarged deployed diameter. Generally for placement ease and the desire to minimize cell trauma, it is preferred to have minimal length change for the device while it is being enlarged in a vessel. Another common problem is that many existing stent designs are limited in their overall flexibility, making stent placement and expansion difficult or impossible in very small tortuous vessels.

It would be desirable to develop a stent that provides all the benefits of previous expandable stent devices while also having controlled shortening properties, excellent flexibility in the delivery and deployed configurations, and/or other desirable properties.

US-A-6,096,072 relates to an expandable stent formed from a plurality of rows of circularly arranged annular members situated side by side in one direction, and connecting rods for connecting the annular members together.

WO 00/42945 relates to an expandable endovascular medical tubular stent which is intended to maintain the walls of anatomical body channels or vessels, the stent being expandable within the vessel by an angioplasty balloon associated with a catheter thereby dilating and expanding the lumen of a vessel. The stent comprises an arrangement of a plurality of radially expandable, serpentine members arranged in interconnected rings. Upon inflation of the balloon, the stent expands in both radial and longitudinal directions in relation to the amount of radially-outwardly directed force by the balloon.

EP 0806190 relates to an angioplasty stent comprising a body constituted by a plurality of successive segments connected in pairs by bridge means so that the successive segments can be oriented relative to one another for the purposes of bending the body in any direction defined by a linear combination of respective orientation axes defined by the bridge connection means.

US 2002/0123796 relates to a stent which provides a folded strut section that provides both structural rigidity and reduction in foreshortening of the stent mechanism. A flexible section provides flexibility for delivery of the stent mechanism. Furthermore, there is a description of flexible section columns angled with respect to each other, and to the longitudinal axis of the stent. These relatively flexible sections are oppositely phased in order to negate any torsion along their length.

A stent, as defined in the preamble of claim 1, is known from EP 0806190.

### SUMMARY OF THE INVENTION

According to a first aspect of the present invention there is provided a stent having a longitudinal axis, comprising:
at least one radial expansion zone oriented essentially perpendicular to the longitudinal axis;
each radial expansion zone comprising at least two undulated expansion elements;
   wherein the undulated radial expansion elements in each radial expansion zone are not attached to each other within the radial expansion zones; further wherein the undulated radial expansion elements are attached to each other outside of the radial expansion zones.

The present invention comprises an improved stent for use in a variety of implantation procedures. The stent of the present invention comprises a series of radial expansion zones oriented essentially perpendicular to the longitudinal axis of the stent. Each of these radial expansion zones comprises at least two expansion elements that are not attached to or otherwise connected with each other within a defined radial expansion zone. Connection between the expansion elements can be provided outside of the radial expansion zones to provide overall stent continuity.

The present invention can be further defined as being a stent having multiple undulated expansion elements arranged around its longitudinal axis. Each of the expansion elements includes a first pitch angle oriented in a step-wise helical fashion around the longitudinal axis and a second pitch angle oriented essentially perpendicular to the longitudinal axis. By orienting the expansion elements relative to each other so that their second pitch angles are aligned with one another within a radial expansion zone, the expansion elements form a virtual radially expandable ring. However, unlike previous discrete ring stent devices, the expansion elements for the stent of the present invention are not connected to one another within the radial expansion zone(s). In this manner, the radial expansion elements are not independently radial expandable from each other.

The stent of the present invention provides a number of improved operating properties over previous stent designs. These include better longitudinally flexibility in both the compacted and expanded configurations, improved expansion characteristics, and controlled length change during expansion.

These and other benefits of the present invention will be appreciated from review of the following description.

### DESCRIPTION OF THE DRAWINGS

The operation of the present invention should become apparent from the following description when considered in conjunction with the accompanying drawings, in which:
Figure 1 is a three-quarter perspective view of one embodiment of a stent of the present invention in its delivery (pre-expanded) configuration;
Figure 2 is a three-quarter isometric view of the stent of Figure 1 shown on a mandrel for clarity in visualizing its stent pattern;
Figure 3 is a planar representation of the stent pattern of the pre-expanded stent of Figure 1;
Figure 4 is an enlarged side elevation view of the stent of Figure 1;
Figure 5 is a three-quarter isometric view of a tranverse section of a single virtual radially expandable ring of the stent of the present invention cut along planes 48A and 48B of Figure 4;
Figure 6 is an exploded three-quarter isometric view of the virtual radially expandable ring of Figure 5;
Figure 7 is a side elevation view of the stent of Figure 1;
Figure 8 is a side elevation view of the stent of Figure 1 shown in its fully expanded state;
Figure 9 is a planar representation of the stent pattern of the fully expanded stent of Figure 8;
Figure 10 is a three-quarter isometric view of a stent of the present invention including a cover on its outer surface;
Figure 11 is a three-quarter isometric view of a stent of the present invention including a coating thereon;
Figure 12 is a planar representation in an unexpanded state of another embodiment of a stent pattern of the present invention employing a single helical element along its length;
Figure 13 is a planar representation in an unexpanded state of another embodiment of a stent pattern of the present invention employing double helical elements along its length;
Figure 14 is a planar representation in an unexpanded state of another embodiment of a stent pattern of the present invention employing quadruple helical elements along its length;
Figure 15 is a planar representation in an unexpanded state of another embodiment of a stent pattern of the present invention employing quintuple helical elements along its length;
Figure 16 is a planar representation in an unexpanded state of a further embodiment of a stent pattern of the present invention employing triple helical elements along its length and modified bridge members;
Figure 17 is a planar representation in an unexpanded state of a further embodiment of a stent pattern of the present invention employing triple helical elements along its length and further modified bridge members;
Figure 18 is a planar representation in an unexpanded state of a further embodiment of a stent pattern of the present invention employing triple helical elements along its length and further modified bridge members;
Figure 19 is a planar representation in an unexpanded state of a further embodiment of a stent pattern of the present invention employing triple helical elements along its length and further modified bridge members;
Figure 20 is a planar representation in an unexpanded state of a further embodiment of a stent pattern of the present invention employing triple helical elements along its length and bridge members that effectively form alternating radial expansion zones.

### DETAILED DESCRIPTION OF THE INVENTION

The present invention is an improved stent device for use in a variety of interventional procedures, such as treatments for coronary artery disease, or other vascular obstructions (e.g., balloon angioplasty and/or stenting), repair of aortic or other vascular aneurysms, creation of various vascular shunts, repair of heart defects, correction of other duct problems in the body, etc. As the term "stent" is used herein, it refers to a device that is adapted to be inserted into a vessel or other passageway or opening within a body and then deployed in place to assist in structurally supporting the host vessel lumen, maintaining patency through the vessel, passageway or opening, and/or to prevent liquids, cells, or other substances from passing through the side wall of the stent, particularly when used with a cover. A stent made in accordance with the present invention may be formed from either plastically deformable material that is expanded in place using a balloon or similar device, or an elastic or springy material that will self-expand in place following placement. Likewise, the stent of the present invention may also be configured to be a permanent implant or erode/resorb over time, incorporate various coatings resulting in a composite structure, and/or comprise a substrate for elution of drugs.

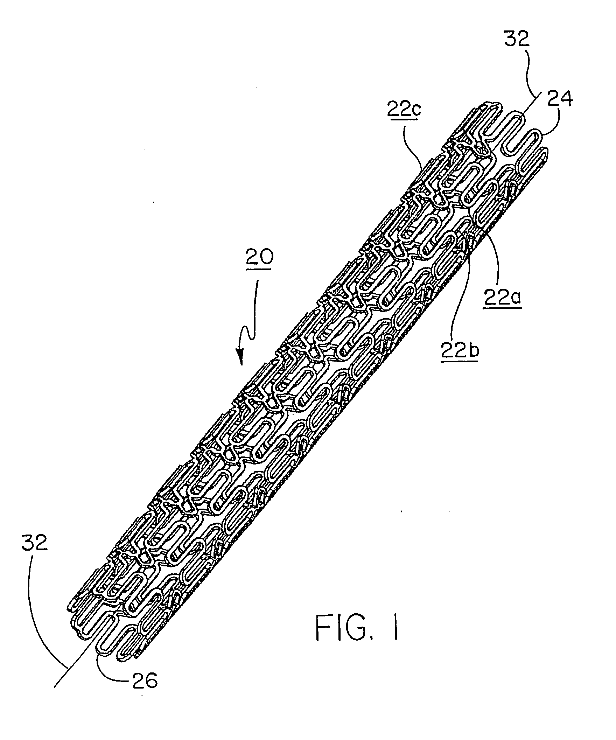

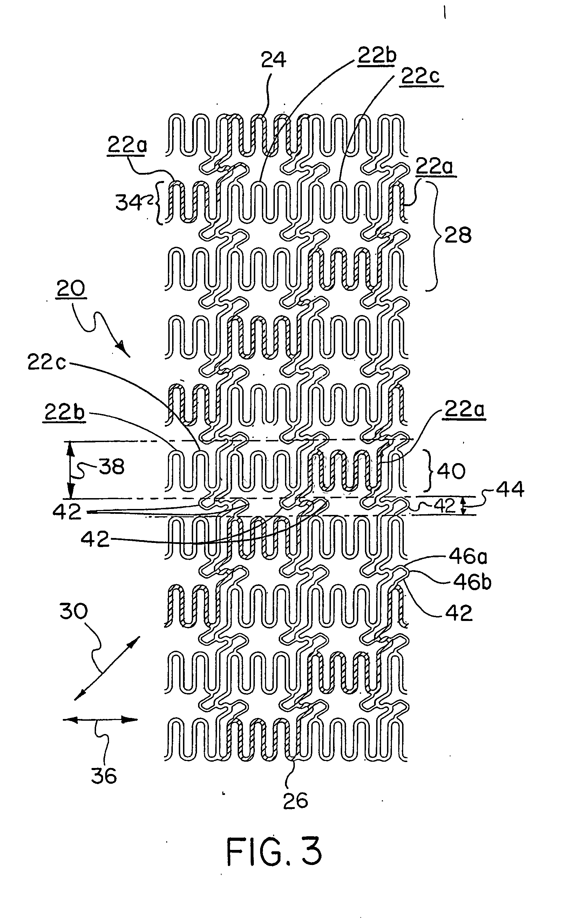

Figures 1 through 8 illustrate one embodiment of a stent 20 of the present invention. In this embodiment the stent 20 is formed from a continuous tube of material that is cut into the desired stent pattern. The stent pattern is one that is a hybrid of previous helical wire patterns and joined-ring patterns. The pattern, best seen in the two-dimensional view of Figure 3, can be defined as having a series of helically disposed expansion elements 22a, 22b, 22c, that each extend from a first end 24 of the stent to a second end 26. Expansion element 22a has been cross-hatched for clarity. In this embodiment each of the expansion elements comprises an undulating pattern, although it is appreciated that any pattern enabling circumferential expansion is feasible with the concepts embodied in the present invention.

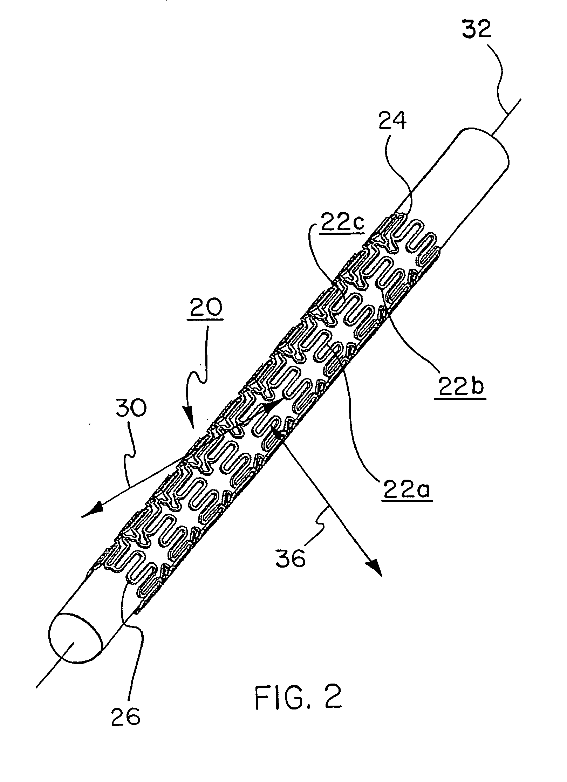

Focusing on only expansion element 22a in Figures 2 and 3, the expansion element 22a takes on a stepped orientation having segments 28 with a first pitch angle 30 extending helically around a longitudinal axis 32 of the stent 20 and segments 34 with a second pitch angle 36 positioned approximately perpendicular to the longitudinal axis 32.

As is shown in Figure 3, by aligning the second pitch angles of each of the expansion elements 22a, 22b, 22c together in a radial expansion zone 38 the multiple expansion elements cooperate to form a virtual radially expandable ring 40. However, unlike previous joined-ring stent designs which are circumferentially continuous, and thus provide a closed cylindrical structure, the expansion elements of the present invention are not attached to each other within the radial expansion zone 38. In this manner, within the radial expansion zone the three expansion elements are separated from each other (i.e., non-continuous), and none of the expansion elements are independently radially expandable (that is, a central radial expansion force applied to the stent within a radial expansion zone will necessarily expand each of the separate expansion elements within the zone at the same time and it is not feasible to expand any one of the expansion elements independently from its adjacent elements). Likewise, the radial expansion zone does not comprise a closed cylindrical structure. This unique feature imparts enhanced flexibility to the present invention in both the expanded and non-expanded configurations.

Connecting bridges 42 are provided in connection zones 44 positioned between (and possibly overlapping) the radial expansion zones 38. The bridges 42 may be constructed to include one or more bends 46a, 46b or other means to provide stored-length therein. The stored-length of the bridges allows the stent to expand radially while not significantly foreshortening in the expansion process. Similarly the bridges can be used to alter the flexural modulus of the stent as well as the degree of endoluminal scaffolding.

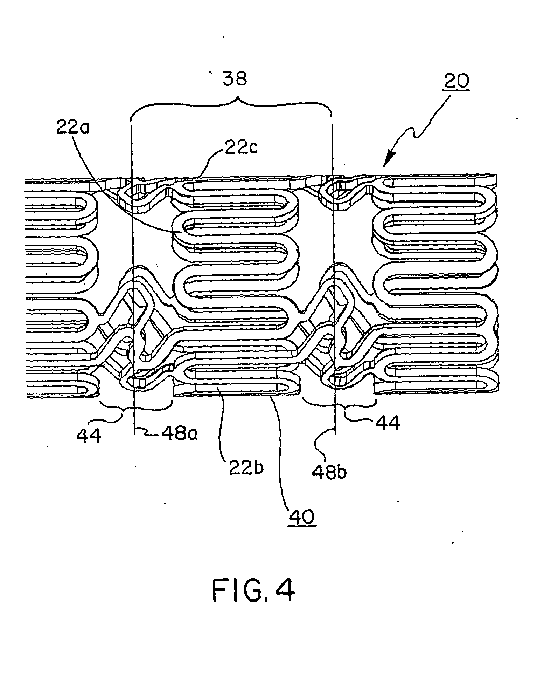

The construction and function of the expansion elements within the radial expansion zone can be better appreciated through review of Figures 4 through 6. Figure 4 illustrates an enlarged view of a radial expansion zone 38 of the present invention comprising expansion elements 22a, 22b, 22c. The radial expansion zone 38 is defined by establishing two sectioning planes 48a, 48b through the mid-point of the connection zones 44 in a manner to establish a virtual radially expandable ring 40 that is symmetrical with adjacent virtual radially expandable rings in the stent 20. In other words, the radial expansion zone is sectioned so as not to encroach onto adjacent virtual radially expandable ring structures. In this manner, pairs of sectioning planes can be periodically applied along the entire length of the device to define the constituent virtual radially expandable rings forming the tubular stent.

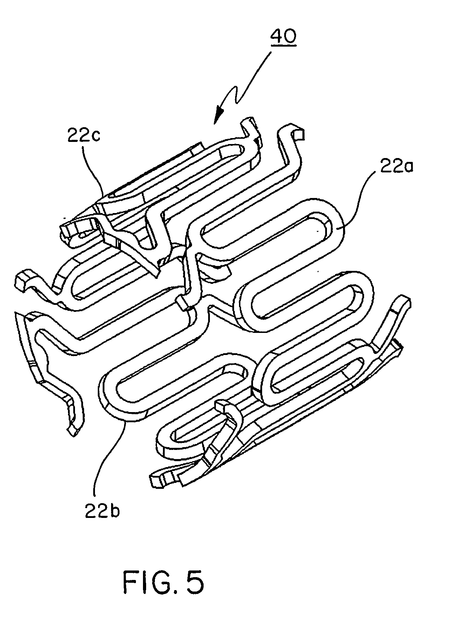

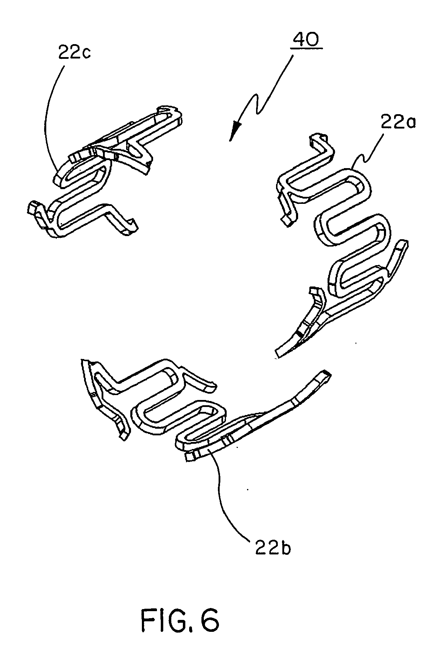

When the radial expansion zone 38 is formed in this manner, the zone 40 can be removed from the rest of the stent 20 structure to form a virtual radially expandable ring 40 as is shown in Figure 5. While this virtual radially expandable ring 40 provides excellent radial force to hold open vessel walls and the like, none of the expansion elements 22a, 22b, 22c in the ring are actually connected together. The lack of interconnectedness among the expansion elements 22 within the radial expansion zone is shown in the exploded view of Figure 6.

It is believed that there are a number of advantages to maintaining separate expansion elements within the radial expansion zone. First, the separation of these elements is believed to contribute to improved stent flexibility in the expanded and non-expanded configurations by facilitating independent movement of the expansion elements within the virtual ring structures. Second, it is believed that the separation of the expansion elements provides more consistent and predictable expansion properties along the entire length of the stent. Third, when combined with the appropriate bridge structures, this design provides for exact engineering of stent foreshortening properties.

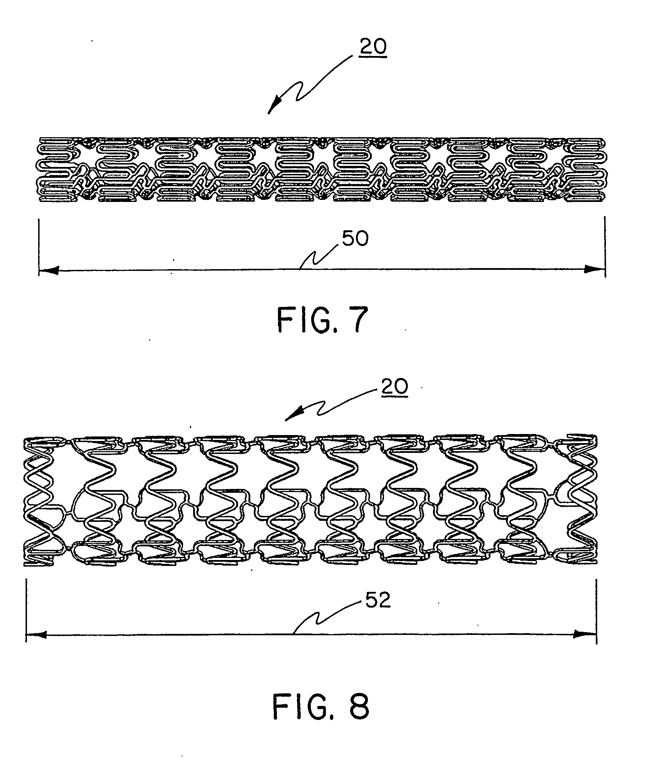

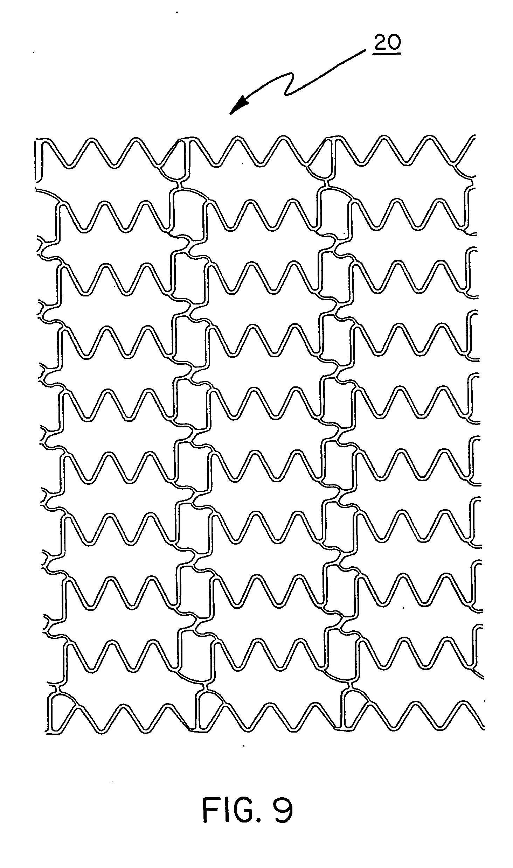

Figures 7 and 8 demonstrate how the stent 20 of the present invention expands from the compacted orientation of Figure 7 to the fully enlarged orientation of Figure 8 with minimal foreshortening of the stent along its length. As can be seen, the compacted stent 20 in Figure 7 has a length 50 that is essentially the same as length 52 of the expanded stent 20 in Figure 8. A two-dimensional representation of the stent 20 as expanded in Figure 8 is illustrated in Figure 9.

Maintaining a consistent overall length of the stent throughout expansion is highly desirable in order to make placement and deployment of the stent more accurate for the medical staff. Additionally, in order to minimize cell irritation or damage during deployment, it is also desirable not to have the stent moving longitudinally during the deployment process. The design of the present invention provides a wide choice of engineering options with respect to stent length change during deployment. In addition to allowing the stent 20 to undergo little or no change in length during deployment, with the design of the present invention it has been determined that by modifying the shape of the expansion elements and the bridges, the stent can be engineered to undergo anything from controlled shortening to even controlled lengthening during expansion.

The stent 20 of the present invention may be formed from a wide variety of materials, including metals (e.g., stainless steel or nitinol), plastics (e.g., PTFE or other fluoropolymers), resorbable materials (e.g., polymers or copolymers possessing one or more of the following monomeric components: glycolide (glycolic acid); lactide (d-lactide, l-lactide, d,l-lactide); trimethylene carbonate; p-dioxanone; caprolactone, hydroxybutyrate, hydroxyvalerate), any other material suitable for implantation, or combinations of any of these or other materials. Additionally, the stent may be provided with additional treatment or therapeutic agents, such a drugs, radiation, radlopaque markers or coatings, or other agents to enhance visualization in-vivo.

To construct the stent of the present invention it is preferred that the stent be cut from a continuous tube of material into the desired pattern, such as through use of a laser. The stent may also be constructed by machining, chemical etching, or other suitable means. The stent may also be formed from a flat sheet of material that is cut into the desired pattern and then bonded together to form a tube having a seam. Finally, although not preferred, the stent of the present invention may be constructed from wires or ribbons that are formed into the desired shapes and then bonded together into the final pattern.

Stents of the present invention can be constructed in a variety of sizes and shapes, including compacted insertion diameters from less than 1 mm to more than 10 mm, and deployed diameters of less than 3 mm to more than 30 mm. It may also be desirable to form stents of the present invention that have tapered or stepped diameters along its length. Stents of the present invention also may be joined together, such as to form a bifurcated stent device, or stent device with a side branch.

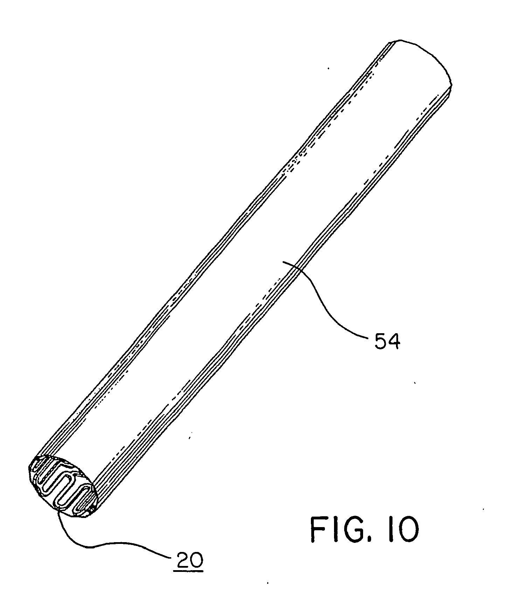

In instances where the stent of the present invention is used to isolate cells, aneurysms, vessel wall defects, and the like, it may be desirable to provide a cover 54 on the stent 20, as is shown in Figure 10. Suitable cover materials include polytetrafluoroethylene (PTFE), expanded PTFE, other fluoropolymers such as fluorinated ethylene propylene (FEP), polyethylene, polypropylene, fluoroelastomer, or a resorbable material. Such covers may be mounted on the stent on its inside, outside, or both over all or a portion of the device length. Additionally, a cover may be provided that allows the stent to be embedded within the cover material, such as through use of a silicone or other elastomeric material. Covers may be coextensive with the length of the stent, as is shown In Figure 10, or they may be either longer or shorter than the stent. Additionally, multiple stents may be provided within a single cover material or multiple covers may be joined together using a stent of the present invention. Further, one or more openings may be provided in the cover material along its length, for instance to accommodate communication with side vessels or similar applications. The cover may be attached to the stent In any suitable manner, including adhesive, friction fit, tape or other tacking material, heat or other bonding techniques, etc. It should be evident from this description that the stent of the present invention may be used with cover materials in any manner now known or later developed without departing from the present invention.

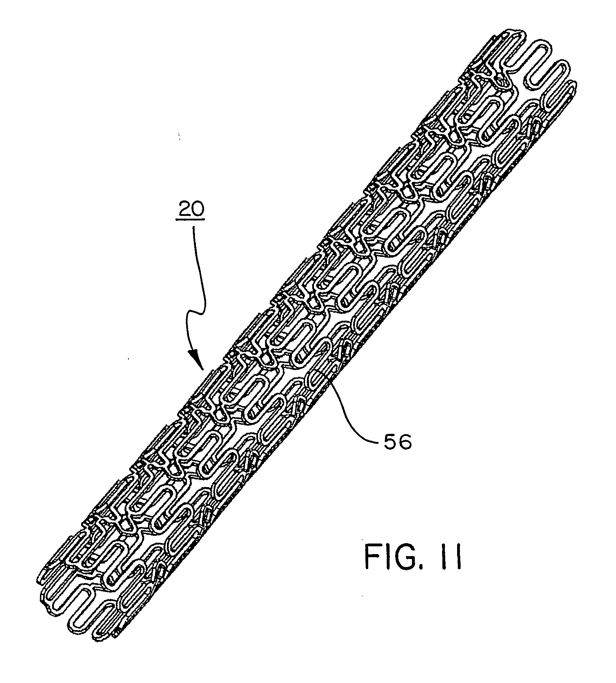

Instead of or in addition to a cover material, the stent of the present invention may include a coating 56 on its surface, as is illustrated in Figure 11. Suitable coating materials may include: fluoroelastomer, ceramic, silicone, polyethylene, carbon, gold, Heparin, hydrogel, or lubricious coatings. Coating materials can provide numerous benefits, including protecting the underlying stent material, providing a substrate for delivery of drugs or other therapeutic substances, isolating the stent material from interaction with surrounding cells, improving fluoroscopic visualization. Coatings can be applied in any material-appropriate manner, such as dip-coating, spray-coating, electro-deposit, or chemical vapor deposition. Additionally, depending upon the application, coatings can be provided to all or only some of the stent surface. Again, coatings can be applied to the stent of the present invention in any form now known or later devised.

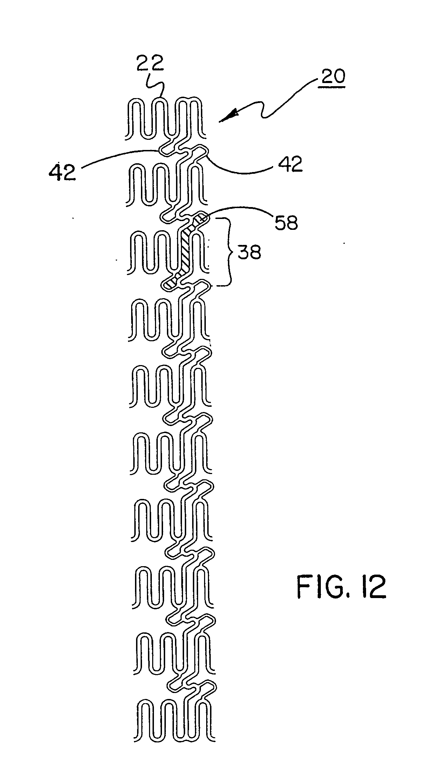

Without departing from the present invention it is possible to modify its stent pattern to provide different stent dimension and/or different stent performance properties. Some of the many permutations of stent designs within the scope of the present invention are illustrated in Figures 12 through 20. Each of these embodiments share the common feature of a repeating series of radial expansion zones 38 arranged along a common longitudinal axis.

Figure 12 illustrates a stent 20 of the present invention that employs a single helical expansion element 22 along its length. The use of bridges 42 in this embodiment attaches the expansion element to itself. As can be seen, this embodiment provides a single expansion cell 58 per radial expansion zone 38. This embodiment is particularly suitable for stents for extremely small diameter applications and/or applications requiring extreme longitudinal flexibility.

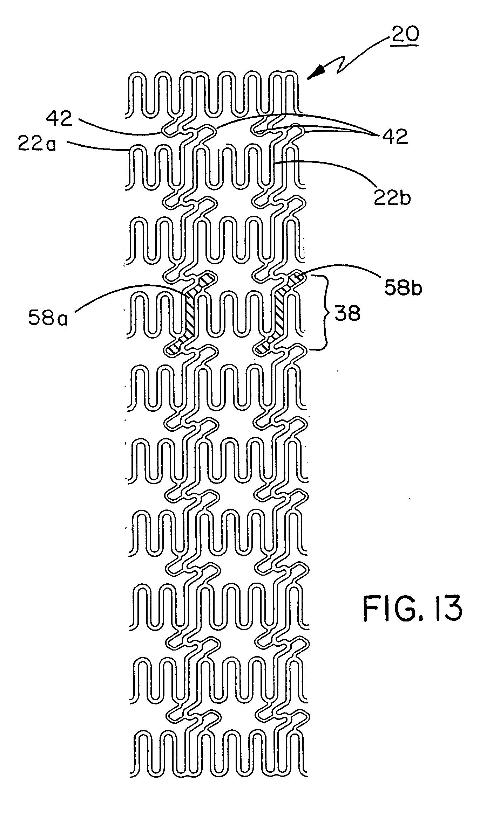

Figure 13 illustrates a stent 20 of the present invention that employs a pair of helical expansion elements 22a, 22b along its length. The bridges 42 join expansion element 22a to expansion element 22b. In this form a pair of expansion cells 58a, 58b is provided per radial expansion zone 38.

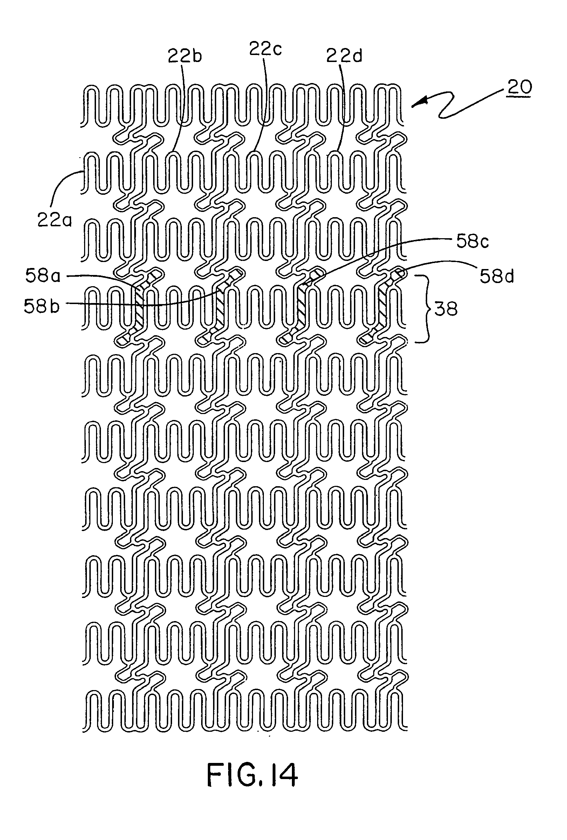

Figure 14 illustrates a larger diameter stent 20 having four expansion elements 22a, 22b, 22c, 22d along its length. This provides four sets of expansion cells 58a, 58b, 58c, 58d per radial expansion zone 38.

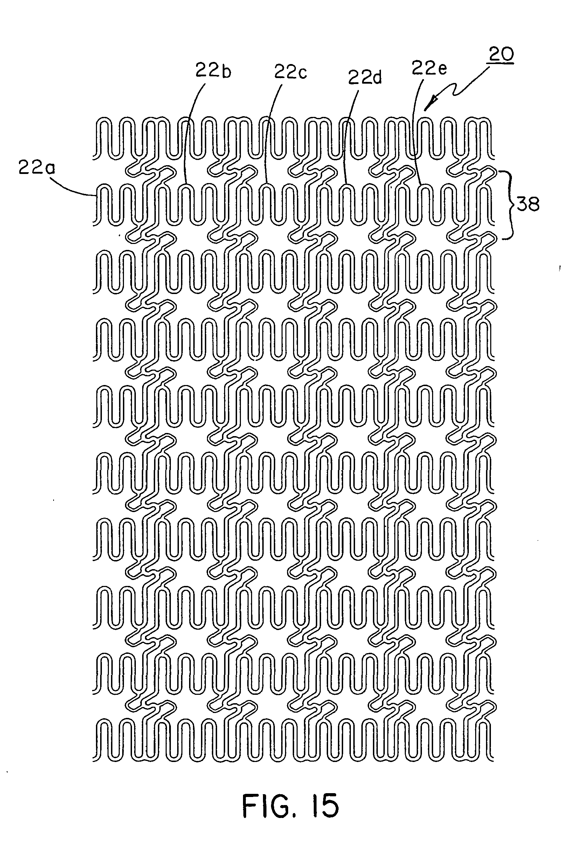

For particularly large diameter applications, Figure 15 illustrates a stent 20 of the present invention that employs five expansion elements 22a, 22b, 22c, 22d, 22e along its length. It should be evident from this description that the number of expansion elements 22 may be increased or decreased to any appropriate number to provide suitable stent performance characteristics.

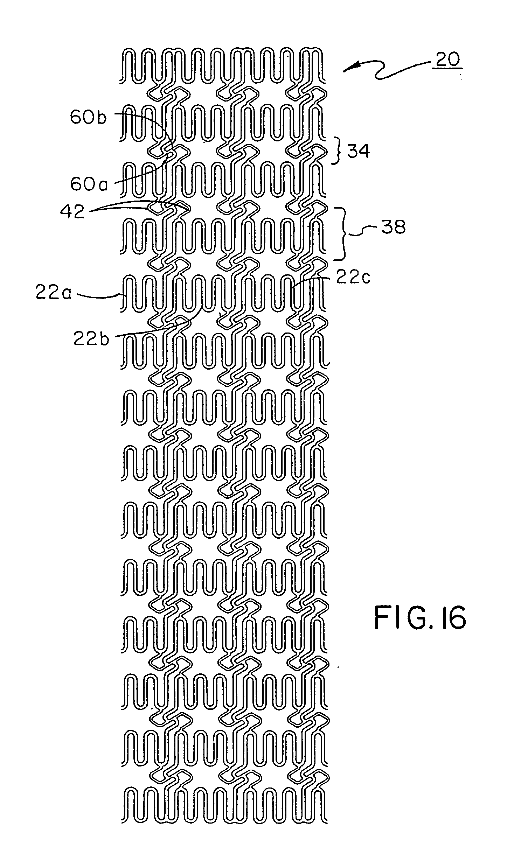

Figure 16 illustrates a stent 20 of the present invention that employs three expansion elements 22a, 22b, 22c with modified bridge 42 structures within the connection zone 44. In this embodiment the bridges 42 do not attach to the expansion elements 22 at a single point, but, rather, attach to the expansion element at two separate attachment points 60a, 60b.

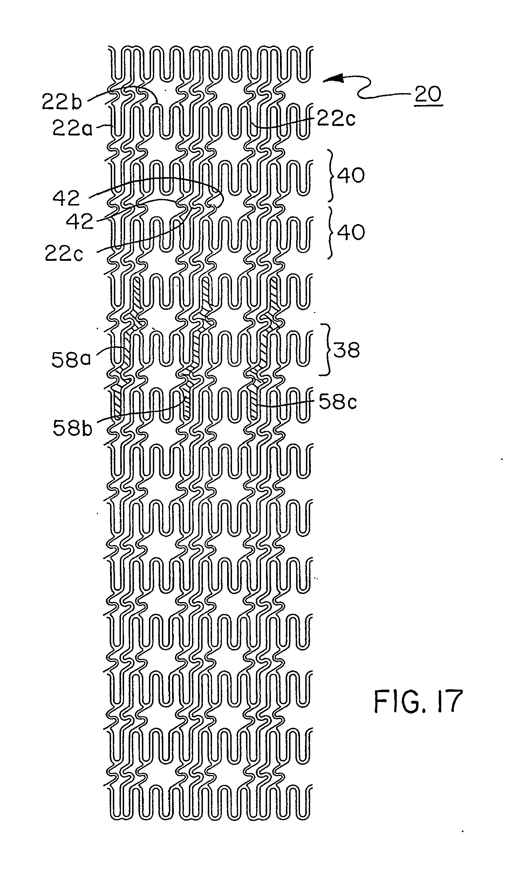

Figure 17 illustrates a stent 20 of the present invention that employs three expansion elements 22a, 22b, 22c with further modified bridge 42 structures within the connection zone 34. In this embodiment the bridges 42 do not connect to the expansion element that passes through connection zone 34, as in the previously described embodiments. Instead, the bridges 42 of this embodiment connect between longitudinally adjacent expansion elements 22 that form the virtual radially expandable ring 40. The stent pattern of this embodiment creates particularly large expansion cells 58a, 58b, 58c that extend over multiple radial expansion zones 38.

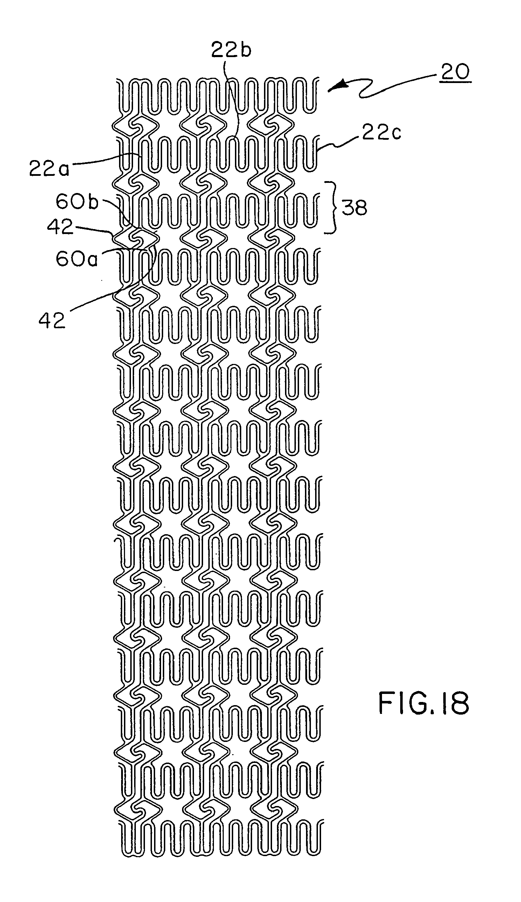

Figure 18 illustrates a stent 20 of the present invention that employs three expansion elements 22a, 22b, 22c and further modified bridge 42 structures. Like the embodiment illustrated in Figure 16, the bridges 42 of this embodiment attach to the expansion elements at separate points 60a, 60b, but are positioned at a greater distance from each other.

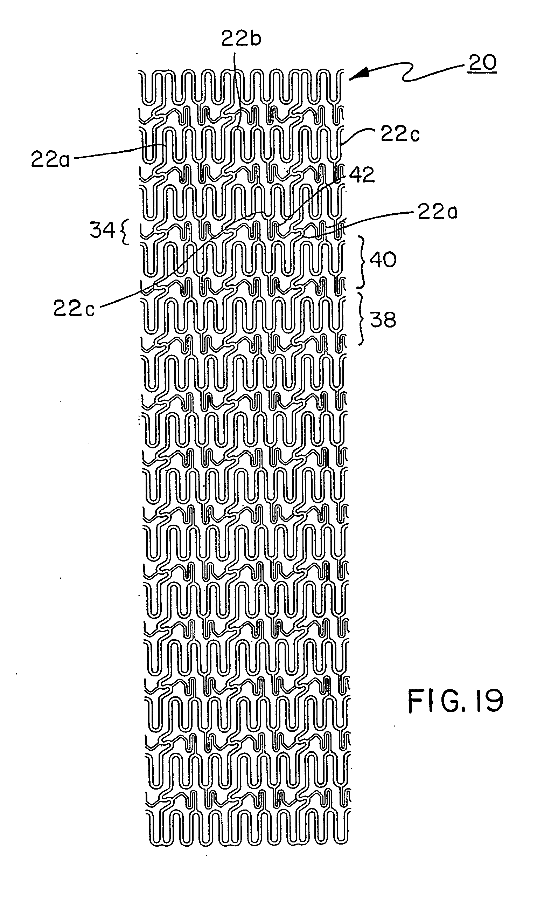

Figure 19 illustrates a stent 20 of the present invention that employs three expansion elements 22a, 22b, 22c and still further modified bridge 42 structures. The bridges 42 in this embodiment attach between the expansion elements 22 (for example, expansion element 22a as designated in the Figure) that pass through connection zone 44 and the expansion elements 22 (for example, expansion element 22c as designated in the Figure) that form the virtual radially expandable ring 40.

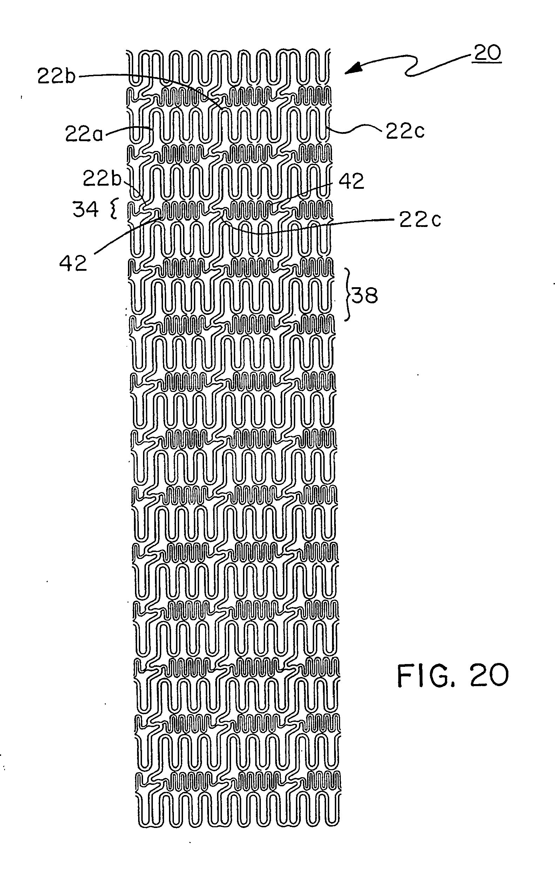

Figure 20 illustrates a stent 20 of the present invention that employs three expansion elements 22a, 22b, 22c and another modification of the bridges 42 structures. The bridges 42 in this embodiment attach directly between the expansion elements 22 that pass through connection zone 44 (for example, between expansion elements 22b and 22c as designated in the Figure). Formed in this manner, the bridges 42 effectively form alternating radial expansion zones with the expansion elements 22.

While particular embodiments of the present Invention have been illustrated and described herein, the present invention should not be limited to such illustrations and descriptions. It should be apparent that changes and modifications may be incorporated and embodied as part of the present invention within the scope of the following claims.

## Claims

1. A stent (20) having a longitudinal axis, comprising:
at least one radial expansion zone (38) oriented
essentially perpendicular to the longitudinal axis; **characterised in that** each radial expansion zone (38) comprises at least
two undulated expansion elements (22a,22b,22c);
wherein the undulated radial expansion elements (22a,22b,22c) in each radial expansion zone (38) are not attached to each other within the radial expansion zones (38); further wherein the undulated radial expansion elements (22a, 22b, 22c) are attached to each other outside of the radial expansion zones (38).

2. The stent (20) of claim 1, wherein the expansion elements (22a,22b,22c) in each radial expansion zone (38) are attached to at least one adjacent undulated expansion element (22a,22b,22c).

3. The stent (20) of claim 1, wherein each of the undulated expansion elements (22a,22b,22c) defines a continuous helical path from a first end (24) of the stent (20) to a second end (26) of the stent (20).

4. The stent (20) of claim 3, wherein the helical path is stepped.

5. The stent (20)of claim 1, wherein a connection zone (44) is defined between adjacent radial expansion zones (38) comprising at least one bridge element (42).

6. The stent (20) of claim 5, wherein the connection zone (44) includes multiple bridge elements (42) therein that attach to one another within the connection zone (44) .

7. The stent (20) of claim 6, wherein the bridge elements (42) attach to one another at intersect points.

8. The stent (20) of claim 5, wherein the connection zone (44) includes multiple bridge elements (42) therein that traverse the connection zone (44).

9. The stent (20) of claim 1, wherein when each of the radial expansion zones (38) is separated from the stent (20) as a whole in an unexpanded state, each of the undulated expansion elements (22a,22b,22c) in the expansion zone (38) will readily separate from one another.

10. The stent (20) of claim 1, wherein the undulated expansion elements (22a,22b,22c) in each of the radial expansion zones (38) are not independently radial expandable from each other.

11. The stent (20) of claim 1, wherein each radial expansion zone (38) includes at least three undulated expansion elements (22a,22b,22c).

12. The stent (20) of claim 1, wherein each radial expansion zone (38) comprises:
multiple undulated elements arranged around the longitudinal axis, each undulating element including a first pitch angle (30) oriented helically around the longitudinal axis and a second pitch angle (36) oriented essentially perpendicular to the longitudinal axis;
wherein the undulating elements are oriented relative to each other so that their second pitch angles (36) are aligned with one another within the radial expansion zone (38), and
wherein the undulating elements are not connected to one another within the radial expansion zone.

13. The stent (20) of claim 12, wherein each undulating element is connected to an adjacent undulating element.

14. The stent (20) of claim 12, wherein the stent (20) includes:
a series of radial expansion zones (38) oriented essentially perpendicular to the longitudinal axis; and
within each radial expansion zone (38) there are at least two separate undulating elements.

15. The stent (20) of claim 14, wherein within each radial expansion zone (38) there are at least three separate undulating elements.

16. The stent (20) of claim 12, wherein when the radial expansion zone (38) is separated from the stent (20) as a whole, each of the undulating elements in the expansion zone (38) will readily separate from one another.

17. The stent (20) of claim 12, wherein the undulating elements in the radial expansion zone (38) are not independently radial expandable from each other.

18. The stent (20) of claim 1, comprising:
a series of at least three defined helical expansion elements (22a,22b,22c) encircling the longitudinal axis;
multiple radial expansion zones (38) within which the expansion elements (22a,22b,22c) cooperate to form a radially expandable ring;
wherein the expansion elements (22a,22b,22c) in the radial expansion zone (38) are not independently radial expandable from each other.

19. The stent (20) of claim 18, wherein:
the stent (20) includes at least one connection zone (44) adjacent to at least one of the radial expansion zones (38); and
wherein the expansion elements (22a,22b,22c) are attached to one another within the connection zone (44).

20. The stent (20) of claim 1, comprising:
a series of at least three defined helical expansion elements (22a,22b,22c) encircling the longitudinal axis; and
multiple radial expansion zones (38) within which the expansion elements (22a,22b,22c) cooperate to form a radially expandable ring.

## Patentansprüche

1. Stent (20) mit einer Längsachse, der Folgendes umfasst:
wenigstens einen radialen Erweiterungsbereich (38), der im Wesentlichen senkrecht zu der Längsachse angeordnet ist, **dadurch gekennzeichnet, dass** jeder radiale Erweiterungsbereich (38) wenigstens zwei wellenförmige Erweiterungselemente (22a, 22b, 22c) umfasst,
wobei die wellenförmigen Erweiterungselemente (22a, 22b, 22c) in jedem radialen Erweiterungsbereich (38) innerhalb der radialen Erweiterungsbereiche (38) nicht aneinander befestigt sind, wobei ferner die wellenförmigen Erweiterungselemente (22a, 22b, 22c) außerhalb der radialen Erweiterungsbereiche (38) aneinander befestigt sind.

2. Stent (20) nach Anspruch 1, wobei die Erweiterungselemente (22a, 22b, 22c) in jedem radialen Erweiterungsbereich (38)an wenigstens einem benachbarten wellenförmigen Erweiterungselement (22a, 22b, 22c) befestigt sind.

3. Stent (20) nach Anspruch 1, wobei jedes der wellenförmigen Erweiterungselemente (22a, 22b, 22c) eine durchgehende spiralförmige Bahn von einem ersten Ende (24) des Stents (20) bis zu einem zweiten Ende (26) des Stents (20) definiert.

4. Stent (20) nach Anspruch 3, wobei die spiralförmige Bahn abgestuft ist.

5. Stent (20) nach Anspruch 1, wobei ein Verbindungsbereich (44) zwischen benachbarten radialen Erweiterungsbereichen (38) definiert wird, der wenigstens ein Brückenelement (42) umfasst.

6. Stent (20) nach Anspruch 5, wobei der Verbindungsbereich (44) mehrere Brückenelemente (42) in demselben einschließt, die innerhalb des Verbindungsbereichs (44) aneinander befestigt sind.

7. Stent (20) nach Anspruch 6, wobei die Brückenelemente (42) an Überschneidungspunkten aneinander befestigt sind.

8. Stent (20) nach Anspruch 5, wobei der Verbindungsbereich (44) mehrere Brückenelemente (42) in demselben einschließt, die den Verbindungsbereich (44) queren.

9. Stent (20) nach Anspruch 1, wobei, wenn jeder der radialen Erweiterungsbereiche (38) in einem nicht erweiterten Zustand von dem Stent (20) als Ganzes getrennt ist, sich alle wellenförmigen Erweiterungselemente (22a, 22b, 22c) in dem Erweiterungsbereich (38) leicht voneinander trennen werden.

10. Stent (20) nach Anspruch 1, wobei die wellenförmigen Erweiterungselemente (22a, 22b, 22c) in jedem der radialen Erweiterungsbereiche (38) nicht unabhängig voneinander in Radialrichtung erweitert werden können.

11. Stent (20) nach Anspruch 1, wobei jeder radiale Erweiterungsbereich (38) wenigstens drei wellenförmige Erweiterungselemente (22a, 22b, 22c) einschließt.

12. Stent (20) nach Anspruch 1, wobei jeder radiale Erweiterungsbereich (38) Folgendes umfasst:
mehrere wellenförmige Elemente, die um die Längsachse angeordnet sind, wobei jedes wellenförmige Element einen ersten Steigungswinkel (30), der spiralförmig um die Längsachse ausgerichtet ist, und einen zweiten Steigungswinkel (36), der im Wesentlichen senkrecht zu der Längsachse ausgerichtet ist, einschließt,
wobei die wellenförmigen Elemente im Verhältnis zueinander so ausgerichtet sind, dass ihre zweiten Steigungswinkel (36) innerhalb des radialen Erweiterungsbereichs (38) miteinander gleichgerichtet sind, und
wobei die wellenförmigen Elemente innerhalb des radialen Erweiterungsbereichs nicht miteinander verbunden sind.

13. Stent (20) nach Anspruch 12, wobei jedes wellenförmige Element mit einem benachbarten wellenförmigen Element verbunden ist.

14. Stent (20) nach Anspruch 12, wobei der Stent (20) Folgendes einschließt:
eine Reihe von radialen Erweiterungsbereichen (38), die im Wesentlichen senkrecht zu der Längsachse ausgerichtet sind, und
wobei es innerhalb jedes radialen Erweiterungsbereichs (38) wenigstens zwei gesonderte wellenförmige Elemente gibt.

15. Stent (20) nach Anspruch 14, wobei es innerhalb jedes radialen Erweiterungsbereichs (38) wenigstens drei gesonderte wellenförmige Elemente gibt.

16. Stent (20) nach Anspruch 12, wobei, wenn der radiale Erweiterungsbereich (38) von dem Stent (20) als Ganzes getrennt ist, sich alle wellenförmigen Elemente in dem Erweiterungsbereich (38) leicht voneinander trennen werden.

17. Stent (20) nach Anspruch 12, wobei die wellenförmigen Elemente in dem radialen Erweiterungsbereich (38) nicht unabhängig voneinander in Radialrichtung erweitert werden können.

18. Stent (20) nach Anspruch 1, der Folgendes umfasst:
eine Reihe von wenigstens drei definierten spiralförmigen Erweiterungselementen (22a, 22b, 22c), welche die Längsachse umschließen,
mehrere radiale Erweiterungsbereiche (38), innerhalb derer die Erweiterungselemente (22a, 22b, 22c) zusammenwirken, um einen in Radialrichtung erweiterbaren Ring zu bilden,
wobei die Erweiterungselemente (22a, 22b, 22c) in dem radialen Erweiterungsbereich (38) nicht unabhängig voneinander in Radialrichtung erweitert werden können.

19. Stent (20) nach Anspruch 18, wobei:
der Stent (20) wenigstens einen Verbindungsbereich (44) angrenzend an wenigstens einen der radialen Erweiterungsbereiche (38) einschließt und
wobei die Erweiterungselemente (22a, 22b, 22c) innerhalb des Verbindungsbereichs (44) aneinander befestigt sind.

20. Stent (20) nach Anspruch 1, der Folgendes umfasst:
eine Reihe von wenigstens drei definierten spiralförmigen Erweiterungselementen (22a, 22b, 22c), welche die Längsachse umschließen, und
mehrere radiale Erweiterungsbereiche (38), innerhalb derer die Erweiterungselemente (22a, 22b, 22c) zusammenwirken, um einen in Radialrichtung erweiterbaren Ring zu bilden.

## Revendications

1. Stent (20), comportant un axe longitudinal, comprenant:
au moins une zone à expansion radiale (38) orientée pour l'essentiel de manière perpendiculaire à l'axe longitudinal; **caractérisé en ce que** chaque zone à expansion radiale (38) comprend au moins deux éléments ondulés à expansion (22a, 22b, 22c) ;
les éléments ondulés à expansion radiale (22a, 22b, 22c) dans chaque zone à expansion radiale (38) n'étant pas fixés les uns aux autres dans la zone à expansion radiale (38) ; et les éléments ondulés à expansion radiale (22a, 22b, 22c) étant fixés les uns aux autres en dehors des zones à expansion radiale (38).

2. Stent (20) selon la revendication 1, dans lequel les éléments d'expansion (22a, 22b, 22c) dans chaque zone à expansion radiale (38) sont fixés sur au moins un élément ondulé à expansion adjacent (22a, 22b, 22c).

3. Stent (20) selon la revendication 1, dans lequel chacun des éléments ondulés à expansion (22a, 22b, 22c) définit une trajectoire hélicoïdale continue, d'une première extrémité (24) du stent (20) vers une deuxième extrémité (26) du stent (20).

4. Stent (20) selon la revendication 3, dans lequel la trajectoire hélicoïdale est étagée.

5. Stent (20) selon la revendication 1, dans lequel une zone de raccordement (44) est définie entre des zones à expansion radiale adjacentes (38), comprenant au moins un élément de liaison (42).

6. Stent (20) selon la revendication 5, dans lequel la zone de raccordement (44) englobe de multiples éléments de liaison (42), fixés les uns aux autres dans la zone de raccordement (44).

7. Stent (20) selon la revendication 6, dans lequel les éléments de liaison (42) sont fixés les uns aux autres au niveau de points d'intersection.

8. Stent (20) selon la revendication 5, dans lequel la zone de raccordement (44) englobe de multiples éléments de liaison (42) traversant la zone de raccordement (44).

9. Stent (20) selon la revendication 1, dans lequel, lorsque chacune des zones à expansion radiale (38) est séparée du stent (20) en tant qu'ensemble, dans un état non expansé, chacun des éléments ondulés d'expansion (22a, 22b, 22c) dans la zone d'expansion (38) peut être séparé facilement des autres éléments.

10. Stent (20) selon la revendication 1, dans lequel les éléments ondulés à expansion (22a, 22b, 22c) dans chacune des zones à expansion radiale (38) ne peuvent pas être expansés radialement de manière indépendante les uns des autres.

11. Stent (20) selon la revendication 1, dans lequel chaque zone à expansion radiale (38) englobe au moins trois éléments ondulés à expansion (22a, 22b, 22c).

12. Stent (20) selon la revendication 1, dans lequel chaque zone à expansion radiale (38) comprend :
de multiples éléments ondulés, agencés autour de l'axe longitudinal, chaque élément ondulé englobant un premier angle de pas (30) orienté de manière hélicoïdale autour de l'axe longitudinal, et un deuxième angle de pas (36) orienté pour l'essentiel de manière perpendiculaire à l'axe longitudinal ;
les éléments ondulés étant orientés les uns par rapport aux autres de sorte que leurs deuxièmes angles de pas (36) sont alignés les uns avec les autres dans la zone à expansion radiale (38) ; et
les éléments ondulés n'étant pas raccordés les uns aux autres dans la zone à expansion radiale.

13. Stent (20) selon la revendication 12, dans lequel chaque élément ondulé est raccordé à un élément ondulé adjacent.

14. Stent (20) selon la revendication 12, dans lequel le stent (20) englobe :
une série de zones à expansion radiale (38), orientées pour l'essentiel de manière perpendiculaire à l'axe longitudinal ; et
chaque zone à expansion radiale (38) contenant au moins deux éléments ondulés séparés.

15. Stent (20) selon la revendication 14, dans lequel chaque zone à expansion radiale (38) contient au moins trois éléments ondulés séparés.

16. Stent (20) selon la revendication 12, dans lequel, lorsque la zone à expansion radiale (38) est séparée du stent (20) en tant qu'ensemble, chacun des éléments ondulés dans la zone à expansion (38) peut être séparé facilement des autres éléments.

17. Stent (20) selon la revendication 12, dans lequel les éléments ondulés dans la zone à expansion radiale (38) ne peuvent pas être expansés radialement de manière indépendante les uns des autres.

18. Stent (20) selon la revendication 1, comprenant :
une série d'au moins trois éléments d'expansion hélicoïdaux définis (22a, 22b, 22c) encerclant l'axe longitudinal ;
de multiples zones à expansion radiale (38) dans lesquelles les éléments d'expansion (22a, 22b, 22c) coopèrent pour former une bague à expansion radiale ;
les éléments d'expansion (22a, 22b, 22c) dans la zone à expansion radiale (38) ne pouvant pas être expansés radialement de manière indépendante les uns des autres.

19. Stent (20) selon la revendication 18, dans lequel :
le stent (20) englobe au moins une zone de raccordement (44) adjacente à au moins une des zones à expansion radiale (38) ; et
les éléments d'expansion (22a, 22b, 22c) sont fixés les uns aux autres dans la zone de raccordement (44).

20. Stent (20) selon la revendication 1, comprenant :
une série d'au moins trois éléments d'expansion hélicoïdaux (22a, 22b, 22c) définis encerclant l'axe longitudinal ; et
de multiples zones à expansion radiale (38), dans lequel les éléments d'expansion (22a, 22b, 22c) coopèrent pour former une bague à expansion radiale.
